# NEW EUROPEAN PATENT SPECIFICATION

(11) **EP 1 948 149 B2**
(45) Date of publication and mention of the opposition decision: **06.07.2022**
(45) Mention of the grant of the patent: 16.11.2011
(21) Application number: 06815305.5
(22) Date of filing: 25.09.2006
(51) Int. Cl.: A61K 31/155, A61K 31/40, A61P 3/10, A61K 9/20, A61K 9/48, A61K 31/4439

(54) **FORMULATION COMPRISING METFORMIN AND VILDAGLIPTIN**
FORMULIERUNG MIT METFORMIN UND VILDAGLIPTIN
NOUVELLE FORMULATION CONTENANT METFORMIN ET VILDAGLIPTIN

(30) Priority: 29.09.2005 US 722624 P
(43) Date of publication of application: 30.07.2008
(73) Proprietor: Novartis AG, 4056 Basel (CH); Novartis Pharma GmbH, 1230 Wien (AT)
(72) Inventor: JOSHI, Yatindra, Princeton, NJ 08540 (US); KOWALSKI, James, Belle Mead, NJ 08502 (US); LAKSHMAN, Jay, Parthiban, Cedar Knolls, NJ 07927 (US); ROYCE, Alan, Edward, Saylorsburg, PA 18353 (US); TONG, Wei-Qin, Basking Ridge, NJ 07920 (US); VASANTHAVADA, Madhav, Basking Ridge, NJ 07920 (US)
(74) Representative: Rouquayrol, Céline Hélène
(86) International application number: PCT/US2006/037198
(87) International publication number: WO 2007/041053

(56) References cited:
- WO-A-2004/110422
- WO-A-2006/047248
- WO-A-2006/078593
- WO-A-2006/135693
- AHREN B ET AL: "Improved meal-related beta-cell function and insulin sensitivity by the dipeptidyl peptidase-IV inhibitor vildagliptin in metformin-treated patients with type 2 diabetes over 1 year" DIABETES CARE, AMERICAN DIABETES ASSOCIATION, ALEXANDRIA, VA, US, vol. 28, no. 8, August 2005 (2005-08), pages 1936-1940, XP002412953 ISSN: 0149-5992
- AHREN B ET AL: "The DDP-4 inhibitor, LAF237, improves glycemic control in patients with type 2 diabetes (T2DM) inadequately treated with metformin" INTERNET CITATION, [Online] June 2004 (2004-06), XP002344251 Retrieved from the Internet: URL:http://test.attendeeinteractive.com/sh ows/ada0401/index.cfm?fuseactio n=Locator.SearchAbstracts> [retrieved on 2005-09-07]
- AHREN BO ET AL: "Prolonged efficacy of LAF237 in patients with type 2 diabetes (T2DM) inadequately controlled with metformin" INTERNET CITATION, [Online] June 2004 (2004-06), XP002344250 Retrieved from the Internet: URL:http://test.attendeeinteractive.com/sh ows/ada0401/index.cfm?fuseactio n=Locator.SearchAbstracts> [retrieved on 2005-09-07]
- PRATLEY R E ET AL: "LONG-TERM EFFICACY OF THE DPP-4 INHIBITOR, LAF237, IN PATIENTS WITH TYPE 2 DIABETES INADEQUATELY TREATED WITH METFORMIN" DIABETOLOGIA, BERLIN, DE, vol. 47, no. SUPPL 1, August 2004 (2004-08), pages A69-A70, XP009061614 ISSN: 0012-186X

## Description

This invention relates to a process for preparing a tablet comprising a dipeptidylpeptidase IV (DPP-IV) inhibitor which is vildagliptin and metformin.

Metformin has been widely prescribed for lowering blood glucose in patients with NIDDM and is marketed in 500, 750, 850 and 1000 mg strengths. However, because it is a short acting drug, metformin requires twice-daily or three-times-daily dosing (500 - 850 mg tab 2-3/day or 1000 mg bid with meals). The biguanide antihyperglycemic agent metformin disclosed in U.S. Patent No. 3,174,901 is currently marketed in the U.S. in the form of its hydrochloride salt (Glucophage@), Bristol-Myers Squibb Company). The preparation of metformin (dimethyldiguanide) and its hydrochloride salt is state of the art and was disclosed first by Emil A. Werner and James Bell, J. Chem. Soc. 121, 1922, 1790-1794. Metformin, can be administered e.g. in the form as marketed under the trademarks GLUCOPHAGE^{™}.

Mefformin, increases the sensitivity to insulin in peripheral tissues of the hosts. Mefformin is also involved in inhibition of glucose absorption from the intestine, suppression of hepatic gluconeogenesis, and inhibition of fatty acid oxidation. Suitable dosage regimens of Mefformin include unit doses of 500 mg two to three time's daily and can even be build up to five times daily or 850 mg once or twice daily. [Martindale, The Complete Drug Reference.

The term "metformin" as employed herein refers to metformin or a pharmaceutically acceptable salt thereof such as the hydrochloride salt, the metformin (2:1) fumarate salt, and the metformin (2:1) succinate salt as disclosed in U.S. application Serial No. 09/262,526 filed March 4, 1999, the hydrobromide salt, the p- chlorophenoxy acetate or the embonate, and other known metformin salts of mono and dibasic carboxylic acids including those disclosed in U.S. Patent No. 3, 174,901, all of which salts are collectively referred to as metformin. It is preferred that the metformin employed herein be the metformin hydrochloride salt, namely, that marketed as GLUCOPHAGE-D or GLUCOPHAGE XR (trademark of Bristol-Myers Squibb Company).

In the present context "a DPP-IV inhibitor", "metformin", "a glitazone", or any specific glitazone like "pioglitazone", "rosiglitazone", is also intended to comprise any pharmaceutically acceptable salt thereof, crystal form, hydrate, solvate, diastereoisomer or enantiomer thereof.

Various DPP-IV inhibitor compounds are described below:
In the present context "a DPP-IV inhibitor" is also intended to comprise active metabolites and prodrugs thereof, such as active metabolites and prodrugs of DPP-IV inhibitors. A "metabolite" is an active derivative of a DPP-IV inhibitor produced when the DPP-IV inhibitor is metabolised. A "prodrug" is a compound that is either metabolised to a DPP-IV inhibitor or is metabolised to the same metabolite(s) as a DPP-IV inhibitor.

DPP-IV inhibitors are known in the art. For example, DPP-IV inhibitors are in each case generically and specifically disclosed e.g. in WO 98/19998,DE19616 486 A1, WO 00/34241, WO 95/15309, WO 01/72290, WO 01/52825, WO 9310127, WO 9925719, WO 9938501, WO 9946272, WO 9967278 and WO 9967279.

The DPP-IV inhibitor used in the process of the invention is (S)-1-[(3-hydroxy-1-adamantyl)amino]acetyl-2-cyanopyrrolidine (LAF237) of formula 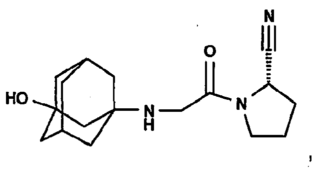 or a pharmaceutical salt thereof.

LAF237 is specifically disclosed in Example 1 of WO 00/34241.

LAF237, and its corresponding pharmaceutically acceptable acid addition salts, may be combined with one or more pharmaceutically acceptable carriers and, optionally, one or more other conventional pharmaceutical adjuvants and administered in the form of tablets.

LAF237, and its corresponding pharmaceutically acceptable acid addition salts, may be formulated into tablets containing an amount of the active substance that is effective for treating conditions mediated by DPP-IV inhibition, such tablets being in unit dosage form and such tablets comprising a pharmaceutically acceptable carrier.

LAF237 may be administered in enantiomerically pure form, e.g., >98%, preferably >99%; or together with the R enantiomer, e.g., in racemic form. The above dosage ranges are based on the compounds of formula (I), excluding the amount of the R enantiomer.

In view of its ability to inhibit DPP-IV, LAF237, and its corresponding pharmaceutically acceptable acid addition salts, is useful in treating conditions mediated by DPP-IV inhibition. Based on the above and findings in the literature, it is expected that the compounds disclosed herein are useful in the treatment of conditions, such as non-insulin-dependent diabetes mellitus, arthritis, obesity, allograft transplantation and calcitonin-osteoporosis. In addition, based on the roles of glucagon-like peptides, such as GLP-1 and GLP-2, and their association with DPP-IV inhibition, LAF237 is useful for example, to produce a sedative or anxiolytic effect, or to attenuate post-surgical catabolic changes and hormonal responses to stress, or to reduce mortality and morbidity after myocardial infarction, or in the treatment of conditions related to the above effects which may be mediated by GLP-1 and/or GLP-2 levels.

More specifically, LAF237, and its corresponding pharmaceutically acceptable acid addition salts, improve early insulin response to an oral glucose challenge and, therefore, are useful in treating non-insulin-dependent diabetes mellitus.

Certain DPP-IV inhibitor compounds are hygroscopic, present stability problems, and are not inherently compactible. Consequently, there is a need to provide a free-flowing, and cohesive composition capable of being compressed into strong tablets with an acceptable *in vitro* dissolution profile and good stability of the active ingredients. Tablets may be defined as solid dosage pharmaceutical forms containing drug substances with or without suitable fillers. They are produced by compression or compaction of a formulation containing the active ingredient and certain excipients selected to aid in the processing and to improve the properties of the product. Tablets may be coated or uncoated and are made from powdered, crystalline materials. They may include various diluents, binders, disintegrants, lubricants, glidants and in many cases, colorants. Excipients used are classified according to the function they perform. For example, a glidant may be used to improve the flow of powder blend in the hopper and into the tablet die.

There has been widespread use of tablets since the latter part of the 19^{th} century and the majority of pharmaceutical dosage forms are marketed as tablets. Major reasons of tablet popularity as a dosage form are simplicity, low cost and the speed of production. Other reasons include stability of drug product, convenience in packaging, shipping and dispensing. To the patient or consumer, tablets offer convenience of administration, ease of accurate dosage, compactness, portability, blandness of taste, ease of administration and elegant distinctive appearance.

Tablets may be plain, film or sugar coated bisected, embossed, layered or sustained-release. They can be made in a variety of sizes, shapes and colors. Tablets may be swallowed, chewed or dissolved in the buccal cavity or beneath the tongue. They may be dissolved in water for local or topical application. Sterile tablets are normally used for parenteral solutions and for implantation beneath the skin.

In addition to the active or therapeutic ingredients, tablets may contain a number of inert materials known as excipients. They may be classified according to the role they play in the final tablet. The primary composition includes a filler, binder, lubricant and glidant. Other excipients which give physical characteristics to the finished tablet are coloring agents, and flavors in the case of chewable tablets. Without excipients most drugs and pharmaceutical ingredients cannot be directly-compressed into tablets. This is primarily due to the poor flow and cohesive properties of most drugs. Typically, excipients are added to a formulation to impart good flow and compression characteristics to the material being compressed. Such properties are imparted through pretreatment steps, such as wet granulation, slugging, spray drying spheronization or crystallization.

Lubricants are typically added to prevent the tableting materials from sticking to punches, minimize friction during tablet compression, and allow for removal of the compressed tablet from the die. Such lubricants are commonly included in the final tablet mix in amounts usually of about 1% by weight.

Other desirable characteristics of excipients include the following:
- High-compressibility to allow strong tablets to be made at low compression forces;
- Impart cohesive qualities to the powdered material;
- Acceptable rate of disintegration
- Good flow properties that can improve the flow of other excipients in the formula; and
- Cohesiveness (to prevent tablet from crumbling during processing, shipping and handling).

There are three commercially important processes for making compressed tablets: wet granulation, direct compression and dry granulation (slugging or roller compaction). The method of preparation and type of excipients are selected to give the tablet formulation the desired physical characteristics that allow for the rapid compression of the tablets. After compression, the tablets must have a number of additional attributes, such as appearance, hardness, disintegrating ability and an acceptable dissolution profile. Choice of fillers and other excipients will depend on the chemical and physical properties of the drug, behavior of the mixture during processing and the properties of the final tablets. Preformulation studies are done to determine the chemical and physical compatibility of the active component with proposed excipients.

The properties of the drug, its dosage forms and the economics of the operation will determine selection of the best process for tableting. Generally, both wet granulation and direct compression are used in developing a tablet.

The dry granulation method may be used where one of the constituents, either the drug or the diluent, has sufficient cohesive properties to be tabletted. The method consists of blending, slugging the ingredients, dry screening, lubrication and compression.

The wet granulation method is used to convert a powder mixture into granules having suitable flow and cohesive properties for tableting. The procedure consists of mixing the powders in a suitable blender followed by adding the granulating solution under shear to the mixed powders to obtain a granulation. The damp mass is then screened through a suitable screen and dried by tray drying or fluidized bed drying. Alternately, the wet mass may be dried and passed through a mill. The overall process includes weighing, dry powder blending, wet granulating, drying, milling, blending lubrication and compression.

In general, powders do not have sufficient adhesive or cohesive properties to form hard, strong granules. A binder is usually required to bond the powder particles together due to the poor cohesive properties of most powders. Heat and moisture sensitive drugs cannot usually be manufactured using wet granulation. The large number of processing steps and processing time are problems due to high level manufacturing costs. Wet granulation has also been known to reduce the compressibility of some pharmaceutical excipients, such as microcrystalline cellulose.

Direct compression is regarded as a relatively quick process where the powdered materials are compressed directly without changing the physical and chemical properties of the drug. The active ingredient(s), direct compression excipients and other auxiliary substances, such as a glidant and lubricant are blended in a twin shell blender or similar low shear apparatus before being compressed into tablets. This type of mixing was believed to be essential in order to prepare "pharmaceutically acceptable" dosage forms. Some pharmaceutical scientists believe that the manner in which a lubricant is added to a formulation must be carefully controlled. Accordingly, lubricants are usually added to a granulation by gentle mixing. It is also believed that prolonged blending of a lubricant with a granulation can materially affect hardness and disintegration time for the resulting tablets. Excessive blending of lubricants with the granulate ingredients can cause water proofing of the granule and reduces tablet hardness or strength of the compressed tablet. For these reasons, high-shear mixing conditions have not been used to prepare direct compression dosage forms.

The advantages of direct compression include uniformity of blend, few manufacturing steps involved, i.e., the overall process involves weighing of powders, blending and compression, hence less cost; elimination of heat and moisture, prime particle dissociation and physical stability.

Pharmaceutical manufacturers would prefer to use direct compression techniques over wet or dry granulation methods because of quick processing time and cost advantages. However, direct compression is usually limited to those situations where the drug or active ingredient has physical characteristics required to form pharmaceutically acceptable tablets. However, one or more excipients must often be combined with the active ingredient before the direct-compression method can be used since many ingredients do not have the necessary properties. Since each excipient added to the formulation increases the tablet size of the final product, manufacturers are often limited to using the direct-compression method in formulations containing a low dose of the active ingredient per compressed tablet.

A solid dosage form containing a high-dose drug, i.e., the drug itself comprises a substantial portion of the total compressed tablet weight, could only be directly compressed if the drug itself has sufficient physical characteristics, e.g., cohesiveness, for the ingredients to be directly compressed and if the drug is properly formulated.

Certain DPP-IV inhibitors are considered high-dose drugs. Most tablet formulations include a range of 70-85% by weight of DPP-IV inhibitor per tablet. This high-dose drug, combined with its rather poor physical characteristics for direct compression, has not permitted direct compression as a method to prepare the final tablet. In addition, the active ingredients have poor stability in presence of water, another factor militating against the use of the wet granulation method.

Another limitation of direct compression as a method of tablet manufacturing is the potential size of the compressed tablets. If the amount of active ingredient is high, a pharmaceutical formulator may choose to wet granulate the active ingredient with other excipients to attain an acceptable sized tablet with the desired amount of active ingredient. The amount of filler, binder or other excipients needed in wet granulation is less than that required for direct compression since the process of wet granulation contributes toward the desired physical properties of the tablet.

Despite the advantages of the direct compression, such as reduced processing time and cost, wet granulation is widely-used in the industry to prepare solid dosage forms. Wet granulation is often preferred over direct compression because wet granulation has a greater chance of overcoming any problems associated with the physical characteristics of various ingredients in the formulation. This provides material which has the required flow and cohesive properties necessary to obtain an acceptable solid dosage form.

The popularity of wet granulation compared to direct compression is based on at least three advantages. First, wet granulation provides the material to be compressed with better wetting properties, particularly in the case of hydrophobic drug substances. The addition of hydrophilic excipients makes the surface of the hydrophobic drug more hydrophilic, reducing disintegration and dissolution problems. Second, the content uniformity of the solid dosage form is generally improved with wet granulation because all of the granules usually contain the same amount of drug. Lastly, the segregation of drug(s) from excipients is avoided.

Segregation could be a potential problem with direct compression. The size and shape of particles comprising the granulate to be compressed are optimized through the wet granulation process. This is because when a dry solid is wet granulated the binder "glues" particles together, so that they agglomerate into spherical granules.

As there is an important amount of metformin present in the tablet prepared by the process of the invention, the size and shape of the resulting tablet is problematic for an easy oral administration to a patient, as well as for an easy tablet manufacturing process which meets all the herein described requirements. Thus there is a need in the industry for techniques and pharmaceutical formulations which will allow manufacturers to prepare high-dose DPP-IV inhibitor and metformin combination tablets (high drug load). The high-dose DPP-IV inhibitor and metformin tablets have to meet all the herein listed requirements with preferably a limited number and amount of pharmaceutical excipients to reduce the size of the tablet.

It is an object of the invention to provide a process for formulating a DPP-IV inhibitor which is vildagliptin or a pharmaceutical salt thereof and metformin into a form of a free-flowing, cohesive tableting powder, capable of being easily granulated or compressed into a tablet.

It is a further object of the invention to provide a process for preparing a high drug load tablet in unit dosage form comprising a DPP-IV inhibitor which is vildagliptin or a pharmaceutical salt thereof and metformin, having an acceptable dissolution profile, as well as acceptable degrees of hardness, friability and resistance to chipping, as well as a proper disintegration time and a high stability of the active ingredients in the tablet.

Vildagliptin is sensitive to moisture and therefore subject to product stability issues i.e. degradation of the active ingredient. In order to overcome this problem the applicant has developed a formulation (with selected excipients) and a direct compression process (to avoid wet granulation) in order to obtain good properties tablets e.g. hardness, friability and with improved stability of the active ingredient, but with only 25% drug load.

Metformin is typically produced by a wet granulation process with a high drug load and is known to be very difficult to process. Roller compaction is also known to be unacceptable due to poor compaction properties and a direct compression process is not recommended for such high drug load formulations. Poor compressibility and tablet friability are known issues and hence were another main emphasis during the development. Other challenges identified are as follows:
- Large amount of Metformin, hence large tablets and low LAF237 drug load.
- Poor processing of Met.
- Met is a wet granulation process and moisture is known to be detrimental to LAF.

Thus there is an unmet need to provide diabetic patients with a compressed tablet comprising between 25 and 100 mg of vilagliptin and up to 1000 mg of metformin with an acceptable tablet size, good tablet properties e.g. hardness, friability and stability of the active ingredients.

It is a further object of the invention to provide a process for preparing a tablet in unit dosage form comprising a DPP-IV inhibitor which is vildagliptin or a pharmaceutical salt thereof and metformin, having a high drug load in order to reduce the size of the tablet wherein the active ingredients remain stable.

It is a further object of the invention to provide a process for preparing a tablet comprising a DPP-IV inhibitor which is vildagliptin or a pharmaceutical salt thereof and metformin, or in any case a salt thereof.

The present invention provides a process for preparing a tablet comprising a DPP-IV inhibitor which is vildagliptin or a pharmaceutical salt thereof and metformin wherein the tableting powder is capable of being compressed into a tablet having adequate size, hardness, stability, rapid disintegration time and an acceptable dissolution pattern.

In addition to the active ingredients, the tableting powder contains a number of inert materials known as excipients. They may be classified according to the role they play in the final tablet. The primary composition includes fillers, binders or diluents, lubricants, disintegrants and glidants. Other excipients which give physical characteristics to the finished tablet are coloring agents, and flavors in the case of chewable tablets. Typically, excipients are added to a formulation to impart good flow and compression characteristics to the material being compressed.

The tablet prepared by the process of this invention comprises the following: the active ingredients which are vildagliptin or a pharmaceutical salt thereof and metformin or a pharmaceutical salt thereof, and a binder.

Examples of pharmaceutically acceptable binders include, but are not limited to, starches; celluloses and derivatives thereof, e.g., microcrystalline cellulose, hydroxypropyl cellulose hydroxylethyl cellulose and hydroxylpropylmethyl cellulose; sucrose; dextrose; corn syrup; polysaccharides; and gelatin. The binder, e.g., may be present in an amount from about 1% to about 40% by weight of the composition preferably 1% to 30% or 1% to 25% or 1 % to 20%.

Optionally, one, two, three or more diluents can be added to the tablet in the process of the invention. Examples of pharmaceutically acceptable fillers and pharmaceutically acceptable diluents include, but are not limited to, confectioner's sugar, compressible sugar, dextrates, dextrin, dextrose, lactose, mannitol, microcrystalline cellulose, powdered cellulose, sorbitol, sucrose and talc. The filler and/or diluent, e.g., may be present in an amount from about 15% to about 40% by weight of the composition. The preferred diluents include microcrystalline cellulose which is manufactured by the controlled hydrolysis of alpha-cellulose, obtained as a pulp from fibrous plant materials, with dilute mineral acid solutions. Following hydrolysis, the hydrocellulose is purified by filtration and the aqueous slurry is spray dried to form dry, porous particles of a broad size distribution. Suitable microcrystalline cellulose will have an average particle size of from about 20 nm to about 200 nm. Microcrystalline cellulose is available from several suppliers. Suitable microcrystalline cellulose includes Avicel PH 101, Avicel PH 102, Avicel PH 103, Avicel PH 105 and Avicel PH 200, manufactured by FMC Corporation. Particularly preferred in the practice of this invention is Avicel PH 102, which has the smallest surface area and pore structure. Preferably the microcrystalline cellulose is present in a tablet formulation in an amount of from about 25% to about 70% by weight. Another preferred range of this material is from about 30% to about 35% by weight; yet another preferred range of from about 30% to about 32% by weight. Another diluent is lactose. Preferably, the lactose is ground to have an average particle size of between about 50 µm and about 500 µm prior to formulating. The lactose is present in the tablet formulation in an amount of from about 5% to about 40% by weight, and can be from about 18% to about 35% by weight, and most preferred, can be from about 20% to about 25% by weight.

Optionally one, two, three or more disintegrants can be added to the tablet in the process of the invention. Examples of pharmaceutically acceptable disintegrants include, but are not limited to, starches; clays; celluloses; alginates; gums; cross-linked polymers, e.g., cross-linked polyvinyl pyrrolidone, cross-linked calcium carboxymethylcellulose and cross-linked sodium carboxymethylcellulose; soy polysaccharides; and guar gum. The disintegrant, e.g., may be present in an amount from about 2% to about 20%, e.g., from about 5% to about 10%, e.g., about 7% about by weight of the composition. A disintegrant is also an optional but useful component of the tablet formulation. Disintegrants are included to ensure that the tablet has an acceptable rate of disintegration. Typical disintegrants include starch derivatives and salts of carboxymethylcellulose. Sodium starch glycolate is the preferred disintegrant for this formulation. Preferably the disintegrant is present in the tablet formulation in an amount of from about 0% to about 10% by weight, and can be from about 1% to about 4% by weight, and most preferred, can be from about 1.5% to about 2.5% by weight.

Optionally one, two, three or more lubricants can be added to the tablet in the process of the invention. Examples of pharmaceutically acceptable lubricants and pharmaceutically acceptable glidants, include, but are not limited to, colloidal silica, magnesium trisilicate, starches, talc, tribasic calcium phosphate, magnesium stearate, aluminum stearate, calcium stearate, magnesium carbonate, magnesium oxide, polyethylene glycol, powdered cellulose and microcrystalline cellulose. The lubricant, e.g., may be present in an amount from about 0.1% to about 5% by weight of the composition; whereas, the glidant, e.g., may be present in an amount from about 0.1% to about 10% by weight. Lubricants are typically added to prevent the tableting materials from sticking to punches, minimize friction during tablet compression and allow for removal of the compressed tablet from the die. Such lubricants are commonly included in the final tablet mix in amounts usually less than 1% by weight. The lubricant component may be hydrophobic or hydrophilic. Examples of such lubricants include stearic acid, talc and magnesium stearate. Magnesium stearate reduces the friction between the die wall and tablet mix during the compression and ejection of the tablets. It helps prevent adhesion of tablets to the punches and dies. Magnesium stearate also aids in the flow of the powder in the hopper and into the die. It has a particle size range of 450-550 microns and a density range of 1.00-1.80 g/mL. It is stable and does not polymerize within the tableting mix. The preferred lubricant, magnesium stearate is also employed in the formulation. Preferably, the lubricant is present in the tablet formulation in an amount of from about 0.25% to about 6%; also preferred is a level of about 0.5% to about 4% by weight; and most preferably from about 0.1% to about 2% by weight. Other possible lubricants include talc, polyethylene glycol, silica and hardened vegetable oils. In an optional embodiment of the invention, the lubricant is not present in the formulation, but is sprayed onto the dies or the punches rather than being added directly to the formulation.

Other conventional solid fillers or carriers, such as, cornstarch, calcium phosphate, calcium sulfate, calcium stearate, magnesium stearate, stearic acid, glyceryl mono- and distearate, sorbitol, mannitol, gelatin, natural or synthetic gums, such as carboxymethyl cellulose, methyl cellulose, alginate, dextran, acacia gum, karaya gum, locust bean gum, tragacanth and the like, diluents, binders, lubricants, disintegrators, coloring and flavoring agents could optionally be employed.

Additional examples of useful excipients which can optionally be added to the tablet in the process of the invention are described in the Handbook of pharmaceutical excipients, 3rd edition , Edited by A.H.Kibbe, Published by: American Pharmaceutical Association, Washington DC, ISBN: 0-917330-96-X, or Handbook of Pharmaceutical Excipients (4th edition), Edited by Raymond C Rowe - Publisher: Science and Practice which are incorporated herewith by reference.

Described is a process for preparing a high drug load tablet or directly compressed tablet, comprising between 80 to 96% by weight on a dry weight basis of active ingredients, wherein the active ingredients consist of vildagliptin and metformin, or in each case a pharmaceutically acceptable salt thereof.

The invention concerns a process according to claim 1, for preparing a tablet.

The process of the invention is for preparing a tablet as described hereinabove, wherein metformin is in the form of granules and wherein the granules contain a binder.

The process of the invention may be for preparing a tablet as described hereinabove, wherein metformin is in the form of granules comprising between 1 to 25% of a binder (1 to 25% of the weight of the granule on a dry weight basis).

The process of the invention may be for preparing a tablet as described herein, obtained by direct compression of the metformin granules with vildagliptin and optionally at least one pharmaceutically acceptable excipient.

The process of the invention may be for preparing a tablet as described hereinabove, comprising between 1 to 20% of a binder preferably between 1 and 12%, between 2.9 and 11% or between 6.5 and 9.5% or between 7.5 and 17.5% or between 12.5 and 17.5% by weight on a dry weight basis of a pharmaceutically acceptable binder.

The process of the invention may be for preparing a tablet as described hereinabove, comprising at least one additional pharmaceutically acceptable excipient which is a lubricant, preferably between 0.1% to 5%, between 0.1% to 2% or between 0.1% to 1.5% by weight of the composition or tablet, or between 0.1% to 1% by weight of the composition or tablet. The process of the invention may be for preparing a tablet as described hereinabove, wherein the lubricant is magnesium stearate.

The process of the invention may be for preparing a tablet as described herein, wherein the binder is selected from starches; celluloses and derivatives thereof, e.g., microcrystalline cellulose, hydroxypropyl cellulose, hydroxylethyl cellulose and hydroxylpropylmethyl cellulose; sucrose; dextrose; corn syrup; polysaccharides; and gelatin

The process of the invention may be for preparing a tablet as described herein, wherein the binder is selected from celluloses and derivatives thereof preferably a hydroxypropylcellulose (HPC).
The herein described ratios have been obtained on a dry weight basis for Vildagliptin, metformin and excipients e.g. the binder.

The process of the invention is for preparing a pharmaceutical tablet as described herein which is in the form of a unit dosage form.

The process of the invention may be for preparing a tablet or pharmaceutical composition comprising as active ingredients,
i) between 0.5 to 35% or between 1.5 to 35%, preferably between 0.5 to 20% or 1.5 to 20% of a DPP-IV inhibitor, which is vildagliptin or a pharmaceutically acceptable salt thereof,
ii) between 65 to 98.5%, preferably between 80 to 98.5% of metformin or a pharmaceutically acceptable salt thereof,
and wherein metformin is in the form of granules comprising between 1 to 25% of a binder (1 to 25% of the weight of the granule on a dry weight basis), or
the process of the invention may be for preparing a high load tablet or high drug load pharmaceutical composition comprising as active ingredients,
i) between 0.5 to 35% or between 1.5 to 35%, preferably between 0.5 to 20% or 1.5 to 20% of a DPP-IV inhibitor, which is vildagliptin or a pharmaceutically acceptable salt thereof,
ii) between 65 to 98.5%, preferably between 80 to 98.5% of metformin or a pharmaceutically acceptable salt thereof,
and wherein metformin is in the form of granules comprising between 1 to 25% of a binder (1 to 25% of the weight of the granule on a dry weight basis).

The process of the invention may be for preparing a tablet as described herein wherein the granules comprise between 1 to 20% preferably between 3 and 13%, between 4.9 and 12% or between 7.5 and 10.5% or between 7.5 and 17.5% or between 12.5 and 17.5% by weight on a dry weight basis of a pharmaceutically acceptable binder.

The process of the invention may be for preparing a tablet as described herein wherein the wherein the binder is selected from starches; celluloses and derivatives thereof, e.g., microcrystalline cellulose, hydroxypropyl cellulose, hydroxylethyl cellulose and hydroxylpropylmethyl cellulose; sucrose; dextrose; corn syrup; polysaccharides; and gelatin.

The process of the invention may be for preparing a tablet as described herein wherein the wherein the binder is selected from celluloses and derivatives thereof, preferably hydroxypropylcellulose (HPC).

The tablets prepared by the process of the invention contain at least one pharmaceutically acceptable excipient.

Additional conventional pharmaceutically acceptable excipients, at least one, e.g. 1, 2, 3 or 4, can optionally be added to the herein described tablets prepared by the process of the invention such as the conventional, binders, diluents, disintegrant, solid fillers or carriers described herein. The formulation does not contain more than 20% or preferably 17.5 or 15% or 11% by weight on a dry weight basis of a pharmaceutically acceptable excipient including the binder.

The process of the invention may be for preparing a tablet as described herein comprising between 1 and 12%, preferably between 2.9 and 11% or between 6.5 and 9.5% or between 7.5 and 17.5% or between 12.5 and 17.5% by weight on a dry weight basis of a pharmaceutically acceptable binder and optionally between 0.1 and 10% by weight on a dry weight basis of a further pharmaceutically acceptable excipient (one, two or more) e.g. between 0.1% to 2% by weight of the composition/tablet of a lubricant (e.g. magnesium stearate). Preferably, the granules comprise between 3 and 13%, between 4.9 and 12% or between 7.5 and 10.5%, or between 7.5 and 17.5% or between 12.5 and 17.5%, by weight on a dry weight basis of a pharmaceutically acceptable binder.

The process of the invention is for preparing a tablet as described herein comprising between 80 to 96% by weight on a dry weight basis of active ingredients, wherein the active ingredients consist of vildagliptin and metformin, or in each case a pharmaceutically acceptable salt thereof.

The process of the invention is for preparing a tablet as described herein comprising at least one additional pharmaceutically acceptable excipient, including a binder.

The process of the invention may be for preparing a tablet as described herein, wherein the additional pharmaceutically acceptable excipient can be fillers, binders or diluents, lubricants, disintegrants and glidants. Other excipients which give physical characteristics to the finished tablet are coloring agents, and flavors in the case of chewable tablets.

The process of the invention may be for preparing a tablet as described herein comprising at least one additional pharmaceutically acceptable excipient which is a lubricant, preferably between 0.1% to 5% or between 0.1% to 2% by weight of the composition, most preferably between 0.5% to 1.5% by weight of the composition/tablet.

The process of the invention may be for preparing a tablet as described herein comprising between 0.1 to 5%, preferably between 0.1 to 2% or 0.5 to 1.5% of magnesium stearate.

The process of the invention may be for preparing a tablet as described herein, wherein the lubricant is magnesium stearate.

The process of the invention is for preparing a tablet as described herein, wherein the metformin granules are produced by melt granulation, with the binder.

The process of the invention is for preparing a tablet as described herein, wherein the metformin granules are produced by melt granulation. Melt granulation processes are described in many publications such as "Hot-melt extrusion Technique": A Review; Iranian Journal of Pharmaceutical Research (2004) 3: 3-16; Rina Chokshi et al. or the review article from Jörg Breitenbach "Melt extrusion: from process to drug delivery technology": European Journal of Pharmaceutics and Biopharmaceutics 54 (2002) 107-117, both incorporated herewith by reference.

The process of the invention is for preparing a tablet as described herein, wherein vildagliptin is present in the form of drug substance.

The process of the invention may be for preparing a tablet as described herein, wherein the DPP-IV inhibitor, which is vildagliptin, represent between 0.5 to 35% or between 1.5 to 35% of the active ingredients i.e. from vildagliptin + metformin.

The process of the invention may be for preparing a tablet as described herein, wherein vildagliptin is in the form of particles and wherein at least 40%, preferably 60%, most preferably 80% even more preferably 90% of vildagliptin has a particle size distribution of less than 250 µm or preferably between 10 to 250 µm or wherein at least 25% or at least 35% of the particle size distribution is between 50 to 150 µm.

The process of the invention may be for preparing a tablet as described herein wherein vildagliptin is in the form of particles.

The process of the invention may be for preparing a tablet as described herein which can additionally be film coated e.g. a film coating of Opadry premix.

The process of the invention may be for preparing a tablet as described herein, wherein the formulation represents one of the layers of a bilayer or trilayer tablet. A preferred bilayer tablet prepared by the process of the invention would contain a first layer comprising a formulation as disclosed herein and a further metformin formulation as a second layer.

Additional conventional pharmaceutically acceptable excipients (at least one, e.g. 1, 2, 3, or 4 excipitents) can optionally be added to the herein described tablets, prepared by the process of the invention, such as the conventional, diluents, disintegrant, solid fillers or carriers described herein. The tablet prepared by the process of the invention does not contain more than 20% by weight on a dry weight basis of a pharmaceutically acceptable excipient including the binder i.e. binder present in the metformin granules.

The process of the invention may be for preparing a tablet which comprises between 0.1 to 5%, preferably between 0.5 to 3% or 0.5 to 1.5% of a pharmaceutically acceptable lubricant, preferably magnesium stearate.

The above described process is for preparing tablet that can comprise one or two diluents selected from microcrystalline cellulose such as Avicel PH 102 and lactose.

In the present application the reference to a pharmaceutically acceptable disintegrant means at least one disintegrant, a mixture of e.g. 2 or 3 disintegrants is also covered.

In the present application the reference to a pharmaceutically acceptable lubricant means at least one lubricant, a mixture of e.g. 2 or 3 lubricants is also covered.

The DPP-IV inhibitor used in the process of the invention is LAF237, preferred diluents are microcrystalline cellulose or lactose or preferably a combination of microcrystalline cellulose and lactose, preferred disintegrant is sodium starch glycolate, preferred binder is a cellulose type binder (celluloses and derivatives thereof) e.g. HPC, and preferred lubricant is magnesium stearate.

The above described formulations are particularly adapted for the production of pharmaceutical tablets e.g. compressed tablets and provides the necessary physical characteristics, dissolution and drug release profiles as required by one of ordinary necessary physical skill in the art. Therefore, the process of the present invention concerns the use of any of the above described formulations, for the manufacture of pharmaceutical tablets according to the process of claim 1.

In particular the tablets obtained with the above described formulations, have very low friability problems, very good breaking strength, improved manufacturing robustness, optimal tablet thickness to tablet weight ratios (direct compressed tablets), less water in the formulation especially directed compressed tablet, good Dispersion Disintegration time DT according to the British Pharmacopoeia 1988, good Dispersion Quality.

This present invention involves blending, granulating and compression. The choice of grades of excipients took also into consideration particle size maintained within a range that allows homogeneity of the powder mix and content uniformity of active ingredients. It prevents segregation of powders in the hopper during compression. The advantages of using the formulation made by the process of the invention is that it imparts compressibility, cohesiveness and flowability of the powder blend. In addition, the compression provides competitive unit production cost, shelf life, eliminates heat and moisture, allows for prime particle dissociation, physical stability and ensures particle size uniformity.

In the development of the herein described process for preparing pharmaceutical compositions, the applicant has discovered that the compressed tablets is particularly advantageous if;
i) the particles comprising Vildagliptin have a particle size distribution of less than 250 µm preferably between 10 to 250 µm, and/or
ii) the water content of the tablet at less than 10% after 1 week at 25°C and 60% room humidity (RH).

Also described is a process for preparing a compressed tablet as described herein, wherein the dispersion contains particles comprising LAF237, in free form or in acid addition salt form, and wherein at least 60%, preferably 80% and most preferably 90% of the particle size distribution in the tablet is less than 250 µm or preferably between 10 to 250 µm.

The present invention concerns a process for preparing a compressed tablet as described herein, wherein the dispersion contains particles comprising LAF237, in free form or in acid addition salt form, and wherein at least 60%, preferably 80% and most preferably 90% of the particle size distribution in the tablet is greater than 10 µm.

The term "wherein metformin is in the form of granules" means that vildagliptin is not present in the granules containing metformin.

The term "wherein at least 60%, preferably 80% and most preferably 90%" means at least 60%, preferably at least 80% and most preferably at least 90%.

The term "wherein at least at least 25%, preferably 35% and most preferably 45%" means at least 25%, preferably at least 35% and most preferably at least 45%.

Further described is a process for preparing a compressed tablet as described herein, wherein the dispersion contains particles comprising LAF237, in free form or in acid addition salt form, and wherein at least 25%, preferably 35% and most preferably 45% of the particle size distribution in the tablet is between 50 to 150 µm.

Further described is a process for preparing a compressed tablet as described herein wherein the dispersion contains particles comprising LAF237, in free form or in acid addition salt form, and wherein;
i) at least 60%, preferably 80% and most preferably 90% of the particle size distribution in the tablet is less than 250 µm preferably between 10 to 250 µm,
ii) the water content of the tablet is less than 10% after 1 week at 25°C and 60% RH.

The process for preparing a compressed tablet as described herein, wherein the dispersion contains particles comprising LAF237, in free form or in acid addition salt form, and wherein;
i) at least 25%, preferably 35% and most preferably 45% of the particle size distribution in the tablet is between 50 to 150 µm,
ii) the water content of the tablet is less than 10% after 1 week at 25°C and 60% RH. pr the water content of the tablet is less than 5% after 1 week at 25°C and 60% RH..

Preferably the LAF237 particles comprise more than 70% of LAF237, most preferably more than 90% or 95% and even more preferably more than 98% of LAF237.

It has been discovered that the selected particle size distribution of DPPIV inhibitor which is LAF237 was particularly important to provide the best compaction of the tablets.

The preferred excipients with an adapted particle size distribution can be picked from e.g. Handbook of Pharmaceutical Excipients (4th edition), Edited by Raymond C Rowe - Publisher: Science and Practice.

Particle size of drug, e.g. LAF237 particles size, is controlled by crystallisazion, drying and/or milling/sieving (non limiting examples are described below). Particle size can also be comminuted using roller compaction and milling/sieving. Producing the right particle size is well known and described in the art such as in "Pharmaceutical dosage forms: volume 2, 2nd edition, Ed.: H.A.Lieberman, L.Lachman, J.B.Schwartz (Chapter 3: SIZE REDUCTION)".

Process to obtain the proper LAF237 particle size is also described in the patent application WO 2005/067976 which is incorporated herein by reference.

Multiple particle sizes have been studied and it has been discovered that the herein described specific size range provides good results for compaction.

PARTICLE SIZE DISTRIBUTION ESTIMATION BY ANALYTICAL SIEVING: Particle size distribution is measured using Sieve analysis, Photon Correlation Spectroscopy or laser diffraction (international standart ISO 13320-1), or electronic sensing zone, light obstruction, sedimentation or microscopy which are procedures well known by the person skilled in the art. Sieving is one of the oldest methods of classifying powders by particle size distribution. Such methods are well known and described in the art such as in any analytical chemistry text book or by the United State Pharmacopeia's (USP) publication USP-NF (2004 - Chapter 786 - (The United States Pharmacopeial Convention, Inc., Rockville, MD)) which describes the US Food and Drug Administration (FDA) enforceable standards. The used techniques are e.g. described in Pharmaceutical dosage forms: volume 2, 2nd edition, Ed.: H.A.Lieberman, L.Lachman, J.B.Schwartz is a good example. It also mentions (page 187) additional methods: Electronic sensing zone, light obstruction, air permeation, sedimentation in gas or liquid.

In an air jet sieve measurement of particle size, air is drawn upwards, through a sieve, from a rotating slit so that material on the sieve is fluidised. At the same time a negative pressure is applied to the bottom of the sieve which removes fine particles to a collecting device. Size analyses and determination of average particle size are performed by removal of particles from the fine end of the size distribution by using single sieves consecutively. See also "Particle Size Measurement", 5th Ed., p 178, vol. 1; T. Allen, Chapman & Hall, London, UK, 1997, for more details on this. For a person skilled in the art, the size measurement as such is thus of conventional character.

Water content of the tablet can be measured using Loss on drying method or Karl-Fischer method which are well known methods to the person skilled in the art (e.g. water content can be measured by loss on drying by thermogrametry). Such methods are well known and described in the art such as in any analytical chemistry text book (J.A. Dean, Analytical Chemistry Handbook, Section 19, McGraw-Hill, New York, 1995) or by the United State Pharmacopeia's (USP) publication USP-NF (2004) which describes the US Food and Drug Administration (FDA) enforceable standards ((2004 - USP - Chapter 921).

This invention provides in particular a process for preparing a compressed tablet or direct compressed tablet which is capable of dispersing in water within a period of 15 to 50 minutes or 20-45 minutes to provide a dispersion which is capable of passing through a sieve screen with a mesh aperture of 710 µm in accordance with the herein defined British Pharmacopoeia test for dispersible tablets.

A tablet prepared by the process of the invention, as well as being quickly dispersible in water, has the added advantage that it meets the British Pharmacopoeia (B.P.) test for dispersible tablets in respect of dispersion times and dispersion quality (i.e. passage through a 710 µ m sieve).

Preferably the dispersion time of a tablet prepared by the process of the invention is less than 15 minutes, more preferably less than 12 minutes and most preferably less than 10 minute.

A further advantage of the tablets prepared by the process of the invention is that because a relatively fine dispersion is formed the tablet will have a lower dissolution time and thus the drug may be absorbed into the blood stream much faster. Furthermore the fast dispersion times and relatively fine dispersions obtained with tablets disclosed herein are also advantageous for swallowable tablets. Thus tablets according to the disclosure can be presented both for dispersion in water and also for directly swallowing. Those tablets according to the disclosure that are intended for swelling are preferably film-coated to aid swallowing.

There is also described a process for preparing a compressed tablet as described herein wherein
i) between 0 and 45 minutes 90 to 99.5 % of LAF237 is released, and
ii) between 10 and 45 minutes 70 to 99 % of metformin is released.

The Paddle method to measure the drug dissolution rate (% of release) is used with 1000ml of 0.01N HCl. Such methods are well known and described in the art such as in any analytical chemistry text book or by the United State Pharmacopeia's (USP) publication USP-NF (2004 - Chapter 711) which describes the US Food and Drug Administration (FDA) enforceable standards.

There is provided a process for preparing a pharmaceutical tablet comprising a DPP-IV inhibitor which is LAF237 or pharmaceutical salts thereof and metformin or pharmaceutical salts thereof, which comprises;
i) granulating metformin and a binder,
ii) drying granules containing metformin and the binder,
iii) blending the DPP-IV inhibitor, which is LAF237, drug substance with the granules containing metformin and the binder,
iv) optionally a lubricant e.g. magnesium stearate is blended with the mixture obtained on step iii),
v) compressing the resulting blend to form tablets in unit dosage form, wherein the granulation of step i) is a melt granulation.

The resulting blend is in the form of a tableting powder, capable of being compressed into a tablet.

The final moisture level of the granulation after drying (LOD) can also be critical in obtaining adequate compaction properties and flow of the Metformin wet granulation (if LOD is to low the compaction properties and tablet friability are poor, while if the LOD is to high the granulation will cause significant picking and/or will begin to form aggregates and restrict powder flow). The proposed target LOD is ∼2% (range of 0.5 to 3.5 preferably a range of 1.5 to 2.4%).

Therefore, in a preferred embodiment during step ii) the granules are dried to an LOD of 0.5-3.5% preferably of 1.5 -2.4%. (LOD: Loos On Drying (method defined in USP)

The granulation of step i) is a melt granulation.

Unexpected good results have been observed if metformin and the binder are granulated by melt granulation (step i)). The obtained final formulations or tablets exhibit the herein described advantages e.g. improved hardness, low friability, good compactibility, dissolution and stability.

Thus in a preferred aspect, metformin and the binder are blended and the blend is passed through an extruder for melt granulation.

Preferably, the extruder is set at between 140 and 220 °C, or between 155 an 205 °C or between 170 and 190°C at mixing zone.

Preferably, the compression step v), is a direct compression of the blend resulting from steps iii) or iv).

In further embodiments, the above described processes can comprise:
- A step i) in order that at the end of step ii) metformin is in the form of granules comprising between 1 to 25% or between 1 to 20% preferably between 1 to 20%, most preferably between 3 and 13%, between 4.9 and 12% or between 7.5 and 10.5% or between 7.5 and 17.5% or between 12.5 and 17.5%by weight on a dry weight basis of a pharmaceutically acceptable binder.
- A step i) wherein at least one further pharmaceutically acceptable excipitent such as a diluent or a disintegrant is added to the mixture to be blended. The further pharmaceutically acceptable excipitent(s) do not represent more than 20% preferably less than 17.5% or 15% by weight on a dry weight basis of the granule weight.
- A step iii) wherein at least one further pharmaceutically acceptable excipitent such as a diluent or a disintegrant is added to the mixture to be blended.
- A further coating step is applied to the resulting compressed core (tablet).
- The compressed cores are optionally dried to an LOD of <1% preferably <0.5% prior to tablet coating.

The DPP-IV inhibitor used in the process of the invention is LAF237, preferred diluents are microcrystalline cellulose or lactose or preferably a combination of microcrystalline cellulose and lactose, preferred disintegrant is sodium starch glycolate, and preferred lubricant is magnesium stearate.

Before step (1) a sieving step is preferably applied to the formulation for basic delumping i.e. to get rid of any agglomerates/cakes. Before step (3) a sieving step is preferably applied to LAF237, before it is added to the metformin granules.

The final product of the process of the invention is in the form of tablets prepared by employing conventional tableting or similar machinery.

A tablet obtained by one of the herein described process which has a hardness comprised between 14 kp and 30 kp at a compression force of 15 kN, and/or a friability between 0.5% and 0.18% at a compression force of 15 to 20 kN,.

The DPP-IV inhibitor is 1-[3-hydroxy-adamant-1-ylamino)-acetyl]-pyrrolidine-2(S)-carbonitrile (LAF237 or vildagliptin) or a pharmaceutical salt thereof.

The dosage of (S)-1-[(3-hydroxy-1-adamantyl)amino]acetyl-2-cyano-pyrrolidine (vildagliptin) is preferably between 10 and 150 mg daily most preferably between 25 and 150 mg or 50 and 100 mg or 25 to 100 mg daily. Preferred examples of daily oral dosage are 25, 30, 35, 45, 50, 55, 60, 70, 80, 90, or 100 mg. The application of the active ingredient may occur up to three times a day, preferably one or two times a day.

The dosage of antidiabetic therapy with metformin should be Individualized on the basis of effectiveness and tolerability while not exceeding the maximum recommended daily dose of metformin which is 2,000 mg. Metformin has been widely prescribed for lowering blood glucose in patients with NIDDM and is marketed in 500, 750, 850 and 1000 mg strengths. However, because it is a short acting drug, metformin requires twice-daily or three-times-daily dosing (500 - 850 mg tab 2-3/day or 1000 mg bid with meals). Preferably the dosage used in the present invention is between 250 and 2000 mg preferably between 250 and 1000 mg. A tablet prepared by the process of the herein described invention may comprise 250 mg, 500 mg, 850 mg or 1000 mg of metformin or a pharmaceutical salt thereof.

There is also described a tablet prepared by the process of the invention, wherein the active ingredients consist of;
i) 50 to 2000 mg of metformin. preferably 250 to 1000mg of metformin
ii) 25 to 100 mg of a DPP-4 inhibitor which is vildagliptin.

Further described is a pharmceutical unit dosage form, which is a tablet, comprising a formulation described here and wherein the active ingredients consist of:
i) 50 to 2000 mg of metformin, preferably 250 to 1000mg of metformin
ii) 25 to 100 mg of a DPP-4 inhibitor which is vildagliptin preferably 25 to 50 mg of vildagliptin.

Further described is a pharmaceutical unit dosage form, which is a tablet, comprising a formulation described here and wherein the active ingredients consist of,
i) 25 mg of vildagliptin and 250 mg of metformin, or in any case a pharmaceutical salt thereof,
ii) 25 mg of vildagliptin and 500 mg of metformin, or in any case a pharmaceutical salt thereof,
iii) 25 mg of vildagliptin and 850 mg of metformin, or in any case a pharmaceutical salt thereof,
iv) 25 mg of vildagliptin and 1000 mg of metformin, or in any case a pharmaceutical salt thereof,
v) 50 mg of vildagliptin and 500 mg of metformin, or in any case a pharmaceutical salt thereof,
vi) 50 mg of vildagliptin and 850 mg of metformin, or in any case a pharmaceutical salt thereof, or
vii) 50 mg of vildagliptin and 1000 mg of metformin, or in any case a pharmaceutical salt thereof.

Also described is a tablet prepared by the process of the invention wherein;
a) the active ingredients consist of;
   i) 25 mg of vildagliptin and 250 mg of metformin, or in any case a pharmaceutical salt thereof,
   ii) 25 mg of vildagliptin and 500 mg of metformin, or in any case a pharmaceutical salt thereof,
   iii) 25 mg of vildagliptin and 650 mg of metformin, or In any case a pharmaceutical salt thereof,
   iv) 25 mg of vildagliptin and 1000 mg of metformin, or In any case a pharmaceutical salt thereof,
   v) 50 mg of vildagliptin and 500 mg of metformin, or In any case a pharmaceutical salt thereof,
   vi) 50 mg of vildagliptin and 850 mg of metformin, or in any case a pharmaceutical salt thereof, or
   vii) 50 mg of vildagliptin and 1000 mg of metformin, or in any case a pharmaceutical salt thereof,
   and
b) metformin is in the form of granules comprising between 1 to 25% of a binder (1 to 25% of the weight of the granule on a dry weight basis), between 1 to 20% of a binder, or between 7.5 and 17.5 % of a binder,
c) the tablet comprises between 80 to 96% by weight on a dry weight basis of active ingredients,
d) the tablet optionally comprises at least one additional excipient such as between 0.1% and 2% magnesium state.

Also described is a tablet prepared by the process of the invention wherein;
- the tablet hardness is comprised between 60 and 340 N,
- the tablet friability is lower than 0.8%, and
- the tablet thickness is comprised between 4.5 and 8.3 mm.

Also described is a tablet prepared by the process of the invention wherein;
- the tablet hardness is comprised between 60 and 340 N,
- the tablet friability is lower than 0.8%,
- the tablet thickness is comprised between 4.5 and 8.3 mm, and
- at lest 70% of vildagliptin is dissolved within 30 minutes
- at least 80% of metformin HCl is dissolved within 45 minutes,
by using the Paddle method.

The tablet prepared by the process of the invention may comprise metformin in the form of its HCl salt.

Any of the herein described tablets prepared according to the claimed process comprise between 80 to 96% by weight on a dry weight basis of active ingredients, wherein the active ingredients consist of vildagliptin and metformin, or in each case a pharmaceutical acceptable salt thereof.

Also described is the use of the herein described tablets, prepared according to the claimed process, for the treatment of conditions, such as non-insulin-dependent diabetes mellitus, arthritis, obesity, allograft transplantation, calcitonin-osteoporosis, Heart Failure, Impaired Glucose Metabolism), IGT (Impaired Glucose Tolerance), neurodegenerative diseases such as Alzheimer's and Parkinson disease, modulating hyperlipidemia, modulating conditions associated with hyperlipidemia or for lowering VLDL, LDL and Lp(a) levels, cardiovascular or renal diseases e.g. diabetic cardiomyopathy, left or right ventricular hypertrophy, hypertrophic medial thickening in arteries and/or in large vessels, mesenteric vasculature hypertrophy, mesanglial hypertrophy, neurodegenerative disorders and cognitive disorders, to produce a sedative or anxiolytic effect, to attenuate post-surgical catabolic changes and hormonal responses to stress, to reduce mortality and morbidity after myocardial infarction, the treatment of conditions related to the above effects which may be mediated by GLP-1 and/or GLP-2 levels.

This invention is further illustrated by the following examples:

### Example 1: Manufacturing process

Due to the Metformin drug substance hardening during storage, a decompaction process using an oscillating mill (Frewitt) fitted with a 1.68mm screen is required. The Metformin is then premixed with HPC-EXF (EXF: Manufacturer's (Aqualon's) grade designation for viscosity and particle size, x=xtrafine. HF=no meaning but viscosity designation that can be compared to other HPC grades, HF, GF, LF, EF) for 1-2 minutes in a high shear mixer. A 9% HPC solution (w/w) is pumped into the high shear granulator at a fixed rate (4 minutes) until adequate granules are formed (total amount of water ∼7%). The granulation is then dried in a fluid bed dryer to a final LOD (loss on drying) of ∼2% (range 1.5 to 2.4%)- The dried granulation is passed through either a Fitzmill (fitted with a 0.078" or 2mm screen) or a Frewitt oscillator (fitted with a 1.68mm screen). The LAF237 drug substance is passed through a 1mm hand screen and blended with the milled Metformin granulation for 300 rotations in a bin blender. The magnesium stearate is also passed through a 1mm hand screen and blended with the Met/LAF mixture for 60 rotations. The blend is then compressed on a rotary tablet press. The compressed cores are dried to an LOD of <0.5% prior to tablet coating. Approximately a 5mg/cm² coating weight is applied during the coating process.

Process parameters used to manufacture batches of the herein described formulations comprising Metformin:LAF237 core batches at 5:1,10:1, 20:1 and 40:1 ratios

| **Manufacturing process steps** | **Process parameter** | **Set point (range)** |
|---|---|---|
| Pre Mixing | Time | 2 minutes |
| Milling | Mesh size | 1.68 or 2.0 mm |
| Granulation + (metformin + binder) | Amount of water | 7% of granulation amount |
| | Rate of addition | 4 minutes (∼200 ml/min) |
| | Kneading time | 2 minutes after water addition |
| | Plough/chopper speed | Low (setting 1) |

| **Manufacturing process steps** | **Process parameter** | **Set point (range)** |
|---|---|---|
| Mixing (LAF237 + (metformin + binder) granules | Time (number of rotations) | 15 minutes (300 rotations) |
| Sieving | Mesh size | 1 mm |
| Final mixing (final blend, including e.g. optional lubricant) | Time (number of rotations) | 3 minutes (60 rotations) |
| Compression | Compression speed | 40 rpm |
| | Compression force | 10 - 23 kN |

Description of manufacturing equipment used for the herein described formulation development

| **Equipment** | **Size/model** | **Unit operation** |
|---|---|---|
| Oscillator | Frewitt | Screening/decompaction |
| High shear mixer | 25 liter Collette Gral | Granulating |
| Convection dryer | GPCG5 Fluid bed | Drying |
| Hammer conventional mill | Fitzmill | Screening |
| Bin or container mixer | 10 and 25 liter container | Blending |
| Tablet press | Manesty Beta | Tabletting |
| Coating pan perforated | Compulab | Coating |

### Batch sizes tested

The batch size for the exploratory batches were typically <1.0 kg. During formulation development, the wet granulation was completed in a 25L Collette Gral mixer with batch sizes ranging from 3.0 to 6.0 kg.

### Statement on the up-scaling potential and robustness of the final process

All process incorporated with the manufacture of the Metformin wet granulation and drying processes as well as the mixing, compression and coating are standard processes and use standard equipment. The FBD (fluid bed dryer) drying process end-point (1.5-2.4%) LOD

Since the moisture level of the dried granulation could have significant impact on tabletting properties, all granulations are preferably prepared using a KG5 mixer and dried in an oven to an LOD of approximately 2% (preferred range 1.5-2.4%).

### Manufacturing process: Alternative

Step 1: Sieve the Metformin and HPC through a 1700µm screen. Place sieved ingredients into a diffusion blender and preblend at 20rpm for 200 rotations.
Step 2: Pass the blend through a twin screw extruder set at 180°C (at mixing zone) - Melt granulation.
Step 3: Sieve the granulation through a 500 µm screen using a frewitt (milling step).
Step 4: Sieve LAF237 through a 500 µm screen and blend with granulation of step 3, at 20 rpm for 300 rotations.
Step 5: Sieve magnesium stearate through a 1000 µm screen and blend at 20 rpm for 60 rotations.
Step 6: Compression of the resulting composition
Step 7: Film coating

### Example 1B: Preparation of metformin granules using the melt granulation process:

| **Ingredient** | **Percentage (w/w)** | **Amount per tablet (mg)** |
|---|---|---|
| Internal phase | | |
| metformin HCl | | 1000 |
| hydroxypropyl cellulose | | 99 |

| External phase | | |
|---|---|---|
| magnesium stearate | | 11 |
| vildagliptin | | 50 |
| Total | | 1160 |

The internal phase ingredients i.e. metformin hydrochloride, and hydroxypropyl cellulose available as KLUCEL EXF from Hercules Chemical Co. (Wilmington, Delaware) are combined and blended in a bin blender for about two hundred rotations. The blend is introduced into the feed section, or hopper, of a twin screw extruder. A suitable twin screw extruder is the PRISM 16 mm pharmaceutical twin screw extruder available from Thermo Electron Corp. (Waltham, Massachusetts).

Located at the end of the twin screw extruder is a die with a bore of approximately three mm. The twin screw extruder is configured with five individual barrel zones, or sections, that can independently adjusted to different parameters. Starting from the hopper to the die, the zones are respectively heated to the following temperatures: 40°C, 110°C, 130°C, 170°C and 185°C. The temperatures of the heating zones do not exceed the melting temperature of metformin hydrochloride which is approximately 232°C. The screw speed is set to 150 rpm, but can be as high as 400 rpm, and the volumetric feed rate is adjusted to deliver between about 30 to 45 grams of material per minute. The throughput rate can be adjusted from 4 g/min to 80 g/min.

The extrudate, or granules, from the extruder are then cooled to room temperature by allowing them to stand from approximately fifteen to twenty minutes. The cooled granules, are subsequently sieved through a 500 micrometer screen (i.e., a one mm screen).

For the external phase, the magnesium stearate is sieved through a 1000 micrometer screen and vildagliptin drug substance is first passed through a 500 micrometer screen. Vildagliptin is then blended with the obtained granules using a suitable bin blender for approximately 150 or 300 rotations. The magnesium stearate is blended with the resulting mixture for 50 or 70 rotations. The resulting final blend is compressed into tablets using a conventional rotary tablet press (Manesty Beta Press) using a compression force ranging between 6kN and 25 kN. The resulting tablets are monolithic and having a hardness ranging from 5 kP to 35 kP. Tablets having hardness ranging from 15 kP to 35 kP resulted in acceptable friability of less than 1.0% w/w after five hundred drops.

Moreover, these tablets have a disintegration time of less than equal to twenty minutes with discs at 37°C in 0.1 N HCl.

### Example 2:

### A. Summary of extended compatibility tests

Excipient compatibility study of the herein described formulations with standard excipients at 50°C/75% (open) for 4 weeks was conducted. Based on the compatibility results, the data indicate that the herein described formulations and tablets provided less degradation of metformin or LAF237.

### B. Stability protocol

Stability studies at 25°C/60%RH, 30/65%RH and 40°C/75%RH was conducted in induction sealed HDPE (high density polyethylene) bottles with desiccant and at 40°C/75%RH open without desiccant (Open). Stability conditions at different time points have shown better result with the herein described formulations and tablets.
RH = relative humidity
(a) Table i) Exploratory formulation stability storage conditions.

| **Storage conditions** | | | | |
|---|---|---|---|---|
| **Interval** | **25°C/60%RH** | **30°C/65%RH** | **40°C/75%RH** | **40°C/75%RH, Open** |
| 3W | | | | X |
| 6W | | | X | X |
| 3M | X | | X | |
| 6M | X | | | |

(b) Table ii) Melt granulation and low moisture series stability storage conditions

| **Storage conditions** | | | | |
|---|---|---|---|---|
| **Interval** | **25°C/60%RH** | **30°C/65%RH** | **40°C/75%RH** | **40°C/75%RH, Open** |
| 3W | | | | X |
| 6W | | | X | X |
| 3M | X | [X] | X | |
| 6M | X | [X] | | |
| 12M | X | [X] | | |
| [ ] = optional test | | | | |

Stability results: Good stability have been obtained with the herein described formulations and tablets.

### Stability of low moisture series formulations (not prepared according to claimed process), Met:LAF 40:1 ratio

(Metformin Directly Compressed) (Pre-granulated material sold as a "new grade" for direct compression in to tablets) + LAF237 (solvent granulation) results in a LAF237 Total degradation of 2.9% in the 40°C/75% RH + 6 weeks closed storage conditions.

(Metformin water granulated with 6.6% of HPC) + LAF237 (solvent granulation) (claimed formulation) results in a LAF237 Total degradation of 0.9% in the 40°C/75% RH + 6 weeks closed storage conditions.

Co-granulation of (metformin + LAF237) with 6.6 % HPC results in a LAF237 Total degradation of 6.6% in the 40°C/75% RH + 6 weeks closed storage conditions.

Furthermore, the applicant has tested many other formulations and has discovered that a formulation, (e.g. tablet in unit dosage form), comprising a DPP-IV inhibitor and metformin, and having a high drug load provides better stability results, especially if a binder is present preferably if HPC is present.

### C. Test conditions for dissolution rate

The method that was selected was based on the results from earlier method development studies showing similar release profiles of Metformin and LAF237 at different pH's (0.01 N HCl, pH 4.5 and pH 6.8 buffer) as well as from paddles or baskets (50 and 100 rpm).
USP Apparatus: I (Baskets)
Rotation Speed: 100 rpm
Dissolution Medium: 0.01 N HCl, degassed.
Volume: 900 ml

The dissolution was performed (n=3) for initial samples only. Dissolution on stability samples have shown good results with the herein described formulations and tablets. The dissolution rate requirements have been met.

### 3. Compositions:

Example of compositions for all dosage strengths are listed in Table 3-1 through Table 3-6

**Table 3-1 Composition at 5:1 ratio for 250/50 mg Met/LAF, film coated tablets**

| **Component** | **Amount per tablet (mg)** | **Weight per weight (%)** | |
|---|---|---|---|
| LAF237 | 50.0 | | 15.3 |
| Metformin HCl | 250.0 | | 76.3 |
| Hydroxypropyl cellulose (Klucel^{®} EXF) | 24.7* | | 7.6 |
| Magnesium stearate | 2.9 | | 0.9 |
| Total core weight | 328.0 | | 100.0 |
| Film coating | | | |
| Opadry premix** | 13.1 | | 4.0 |
| Purified water, USP | q.s.^{a} | | |
| Total film coated tablet weight | 341.0 | | |
| | | | |
| LAF237 | 50.0 | 15.24 | |
| Metformin HCl | 250.0 | 76.22 | |
| Hydroxypropyl cellulose (Klucel^{®} EXF) | 24.75* | 7.6 | |
| Magnesium stearate | 3.25 | 0.99 | |
| Total core weight | 328.0 | 100.0 | |
| Film coating | | | |
| Opadry premix** | 12.0 | 3.53 | |
| Purified water, USP | q.s.^{a} | | |
| Total film coated tablet weight | 340.0 | | |

| | | | |
|---|---|---|---|
| ^{a} Removed during processing. * 9% (w/w) calculated based on total quantity Metformin HCl and HPC. | | | |

**Table 3-2 Composition at 10:1 ratio for 250/25 mg and 500/50 mg Met/LAF, film coated tablets**

| **Component** | **250/50 mg amount per tablet (mg)** | **250/50 mg weight per weight (%)** | **500/50 mg amount per tablet (mg)** | **500/50 mg weight per weight (%)** |
|---|---|---|---|---|
| LAF237 | 50.0 | 8.3 | 50.0 | 8.2 |
| Metformin HCl | 250.0 | 82.7 | 500.0 | 82.7 |
| Hydroxypropyl cellulose (Klucel^{®} EXF) | 24.7* | 8.2 | 49.5* | 8.2 |
| Magnesium stearate | 2.7 | 0.9 | 5.4 | 0.9 |
| Total core weight | 302.0 | 100.0 | 605.0 | 100.0 |
| Film coating | | | | |
| Opadry premix** | 12.1 | 4.0 | 24.2 | 4.0 |
| Purified water, USP | q.s.^{a} | | q.s.^{a} | |
| Total film coated | 315.0 | | 629.0 | |
| tablet weight | | | | |
| | | | | |
| LAF237 | 50.0 | 15.24 | 50.0 | 8.25 |
| Metformin HCl | 250.0 | 76.22 | 500.0 | 82.51 |
| Hydroxypropyl cellulose (Klucel^{®} EXF) | 24.75* | 7.55 | 49.5* | 8.17 |
| Magnesium stearate | 3.25 | 0.99 | 6.5 | 1.07 |
| Total core weight | 328.0 | 100.0 | 606.0 | 100.0 |
| Film coating | | | | |
| Opadry premix** | 12 | 3.52 | 18 | 2.89 |
| Purified water, USP | q.s.^{a} | | q.s.^{a} | |
| Total film coated tablet weight | 340.0 | | 624.0 | |

| | | | | |
|---|---|---|---|---|
| a Removed during processing. * 9% (w/w) calculated based on total quantity Metformin HCl and HPC. | | | | |

**Table 3-3 Composition at 17:1 ratio for Met/LAF 850/50 mg, film coated tablets**

| **Component** | **amount per tablet (mg)** | **weight per weight (%)** | |
|---|---|---|---|
| LAF237 | 50.0 | | 5.0 |
| Metformin HCl | 850.0 | | 85.6 |
| Hydroxypropyl cellulose (Klucel^{®} EXF) | 84.1* | | 8.5 |
| Magnesium stearate | 8.9 | | 0.9 |
| Total core weight | 993.0 | | 100.0 |
| Film coating | | | |
| Opadry premix** | 39.7 | | 4.0 |
| Purified water, USP | q.s.^{a} | | |
| Total film coated tablet weight | 1033.0 | | |
| | | | |
| LAF237 | 50.0 | 5.03 | |
| Metformin HCl | 850.0 | 85.51 | |
| Hydroxypropyl cellulose (Klucel^{®} EXF) | 84.15* | 8.47 | |
| Magnesium stearate | 8.85 | 0.99 | |
| Total core weight | 994.0 | 100.0 | |
| Film coating | | | |
| Opadry premix** | 26.0 | | |
| Purified water, USP | q.s.^{a} | | |

| **Component** | | **amount per tablet (mg)** | **weight per weight (%)** |
|---|---|---|---|
| Total film coated tablet weight | | 1020.0 | |

| | | | |
|---|---|---|---|
| ^{a} Removed during processing. * 9% (w/w) calculated based on total quantity of Metformin HCl and HPC. | | | |

**Table 3-4 Composition at 20:1 ratio for Met/LAF 500/25 mg and 1000/50 mg, film coated tablets**

| **Component** | **500/25 mg amount per tablet (mg)** | **500/25 mg weight per weight (%)** | **1000/50 mg amount per tablet (mg)** | **1000/50 mg weight per weight (%)** |
|---|---|---|---|---|
| LAF237 | 25.0 | 4.3 | 50.0 | 4.3 |
| Metformin HCl | 500.0 | 86.3 | 1000.0 | 86.3 |
| Hydroxypropyl cellulose | 49.5* | 8.5 | 98.9* | 8.5 |
| Hydroxypropyl (Klucel^{®} EXF) Magnesium stearate | 5.2 | 0.9 | 10.4 | 0.9 |
| Total core weight | 580.0 | 100.0 | 1159.0 | 100.0 |
| Film coating | | | | |
| Opadry premix** | 23.2 | 4.0 | 46.4 | 4.0 |
| Purified water, USP | q.s.^{a} | | q.s.^{a} | |
| Total film coated tablet weight | 603.0 | | 1206.0 | |
| | | | | |
| LAF237 | 25.0 | 4.31 | 50.0 | 43.1 |
| Metformin HCl | 500.0 | 86.21 | 1000.0 | 86.21 |
| Hydroxypropyl | 49.5* | 8.53 | 99* | 8.53 |
| cellulose (Klucel^{®} EXF) | | | | |
| Magnesium | 5.5 | 0.95 | 11 | 0.95 |
| stearate | | | | |
| Total core weight | 580.0 | 100.0 | 1160.0 | 100.0 |
| Film coating | | | | |
| Opadry premix** | 18 | | 28 | 2.36 |
| Purified water, USP | q.s.^{a} | | q.s.^{a} | |
| Total film coated | 598 | | 1188 | |
| tablet weight | | | | |
| ^{a} Removed during processing. | | | | |
| * 9% (w/w) calculated based on total quantity Metformin HCl and HPC. | | | | |

**Table 3-5 Composition at 34:1 ratio for Met/LAF 850/25 mg, film coated tablets**

| **Component** | **amount per tablet (mg)** | **weight per weight (%)** |
|---|---|---|
| LAF237 | 25.0 | 2.6 |
| Metformin HCl | 850.0 | 87.8 |
| Hydroxypropyl cellulose (Klucel^{®} EXF) | 84.1* | 8.7 |
| Magnesium stearate | 8.7 | 0.9 |
| Total core weight | 968.0 | 100.0 |
| Film coating | | |
| Opadrv premix** | 38.7 | 4.0 |
| Purified water, USP | q.s.^{a} | |
| Total film coated tablet weight | 1006.0 | |
| ^{a} Removed during processing. | | |
| * 9% (w/w) calculated based on total quantity of Metformin HCl and HPC. | | |

| *Table 3-6 Composition at 40:1 ratio for Met*/*LAF 1000*/*25 mg, film coated tablets* | | |
|---|---|---|
| **Component** | **amount per tablet (mg)** | **weight per weight (%)** |
| LAF237 | 25.0 | 2.2 |
| Metformin HCl | 1000.0 | 88.2 |
| Hydroxypropyl cellulose (Klucel^{®} EXF) | 98.9* | 8.7 |
| Magnesium stearate | 10.2 | 0.9 |
| Total core weight | 1134.0 | 100.0 |
| Film coating | | |
| Opadry premix** | 45.4 | 4.0 |
| Purified water, USP | q.s.^{a} | |
| Total film coated tablet weight | 1179.0 | |
| ^{a} Removed during processing. | | |
| * 9% (w/w) calculated based on total quantity of Metformin HCl and HPC. | | |

### Example 4: The tablets prepared in accordance with the above Description and examples can be tested as follows.

### Tablet Evaluation Methods

1. Average tablet weight. Twenty tablets are weighed on an analytical balance and the average tablet weight calculated.
2. Tablet breaking strength (kilo bond-kp). tablets are individually tested using a Schleuniger crushing strength tester, and the average breaking strength calculated.
3. Friability (% loss). 10 tablets, accurately weighed, are subjected to 10 minutes friability testing using a Roche Friabilator. The tablets are dedusted, reweighed, and the weight loss due to the friability is calculated as a percentage of the initial weight.
4. Dispersion Disintegration time DT (The test for dispersible tablets defined in the British Pharmacopoeia, 1988, Volume II, page 895 - BP 1988). Tablets are tested in accordance to the above-defined BP test (without discs) for dispersible tablets. This utilizes water at a temperature of 19°- 21 °C.
5. Dispersion Quality. In accordance with the BP uniformity of dispersion test for dispersible tablets (BP 1988 Volume II page 895), two tablets are placed in 100 ml of water at 19°-21°C. and allowed to disperse.

### Granule Evaluation Methods

1. Loss on Drying (LOD). The residual moisture content of the granule (LOD) can be determined on a 3-4 g sample using a Computrac moisture analyser set at 90° C. operated in accordance with the manufacturer's procedure.
2. Weight Median Diameter (WMD). A 10 g sample of granule is sifted for 2 minutes at suitable pulse and sift amplitudes in an Allen Bradley sonic sifter in accordance with manufacturer's instructions. Sieves of 300 µm, 250 µm, 200 µm, 150 µm, 100 µm, 53 µm and 40 µm are used. The WMD is calculated from the cumulative percentage undersize size distribution using a computer program.

### Example 5:

### Improved manufacturing robustness

A preliminary compactibility assessment is carried out on a Carver press using different formulations.

Data demonstrate that our claimed compositions on being compressed with increasing levels of pressure (compression force) show well adapted tablet strength. In particular e.g. the herein described formulations have shown a good tablet strength and compactibility. With increasing pressure (compression force) our claimed formulations and selected ranges show a substantially useful increase in tablet strength.

A compactibility study (D. Becker, personal communication) is carried out on an instrumented Korsch single station press with force and displacement sensors on both upper and lower punches.

A clear indication is afforded from these data that LAF237 tablets are very likely to have poor tablet hardness/crushing strength unless diluted out using sufficient filler with excellent compactibility. However, our claimed formulations and selected ranges are particularly adapted to provide the required compactibility especially for the LAF237:metformin ratio of 1:5.

The results obtained show that convenient tablet hardness can be obtained if the metformin granules contain e.g. between 1 and 20% preferably between 3 and 13%, between 3 and 17.5% of a binder such as HPC.

### Example 6: Friability

Evaluation is carried out using a Manesty Betapress at 6 different settings: strain rate settings of 66-90 rpm (63,000-86,000 TPH) and force of 7.5-15 kN. The trials uses Flat-faced Beveled-edge (FFBE) tooling of 9 mm diameter for 250 mg tablets and 10 mm diameter for 310 mg tablets (other diameters are used depending on the weight of the tested tablet). Friability, Compression profile, Strain rate profile and Weight variation are the measured outcomes. Study design and the friability results obtained from the study are used to determine the variables (particle size distribution in the formulation, tablet weight, tablet thickness and weight, water content in the tablet etc) impacting the outcome of hardness. Our claimed formulations and selected ranges are particularly adapted to provide the required Friability.

Example - Tablets having a Metformin:LAF237 ratio of 20:1 : The results show that tablets comprising LAF237 + (metformin granules without binder) have around 0.8% friability, while tablets comprising LAF237 + (metformin granules comprising 12% HPC) have less than 0.2% friability (at a compression force of 15kN).

### Example 7: Mechanical stress (particle size distribution)

The material in the desired particle size range can be produced from any form of vildagliptin e.g. amorphous vildagliptin, by mechanical stress. This stress can be mediated by impact, shear or compression. In most commercially available grinding equipment a combination of these principles occurs. For vildagliptin preferably a mechanical impact or jet mill is used. The most preferable mechanical impact mill can be equipped with different kind of beaters, screens, liners or with pin plates. For our process preferably an impact mill with plate beater and a slit screen 5 * 2.5 cm is used. The impact speed should be variable between 20 and 100 m/s (as peripheral speed) to adapt to any batch to batch variation. In our case a peripheral speed of the beater of about 40 - 50 m/s is used.

## Claims

1. A process for preparing a pharmaceutical tablet comprising between 80 to 96% by weight on a dry weight basis of active ingredients, wherein the active ingredients consist of a DPP-IV inhibitor which is vildagliptin or a pharmaceutical salt thereof and metformin or in any case a pharmaceutical salts thereof, which comprises;
i) granulating metformin and a binder,
ii) drying granules containing metformin and the binder,
iii) blending the DPP-IV inhibitor, drug substance which is vildagliptin or a pharmaceutical salt thereof with the granules containing metformin and the binder,
iv) optionally a lubricant e.g. magnesium stearate is blended with the mixture obtained on step iii),
v) compressing the resulting blend to form tablets in unit dosage form,
wherein the granulation of step i) is a melt granulation.

2. A process according to claim 1, wherein during step ii) the granules are dried to an LOD of 0.5-3.5% preferably of 1.5 - 2.4%.

3. A process according to any of claims 1 to 2, wherein at the end of step ii) metformin or a pharmaceutical salt thereof, is in the form of granules comprising between 1 to 25% or between 3 and 13% or between 4.9 and 12% or
between 7.5 and 10.5%, or between 7.5 and 17.5% or between 12.5 and 17,5% by weight on a dry weight basis of a pharmaceutically acceptable binder.

4. A process according to any of claims 1 to 3, wherein at least one further pharmaceutically acceptable excipitent is added to the mixture to be blended during step i) or during step iii).

5. A process according to claim 4, wherein the further pharmaceutically acceptable excipitent is a diluent or a desintegrant.

6. A process according to any of claims 2 to 5, wherein a further coating step is applied to tablet resulting from step v).

7. A process according to any of claims 1 to 6, comprising a granulation step i) wherein metformin and the binder are blended and the blend is passed through an extruder for melt granulation.

8. A process according to claim 7, wherein the extruder is set at between 140 and 220 °C, or between 155 an 205°C or between 170 and 190 °C at mixing zone.

9. A process according to any of claims 2 to 8, wherein the binder is a cellulose or derivative thereof, selected from microcrystalline cellulose, hydroxypropyl cellulose, hydroxylethyl cellulose and hydroxylpropylmethyl cellulose.

## Patentansprüche

1. Verfahren zur Herstellung einer pharmazeutischen Tablette, die zwischen 80 bis 96 Gew.-% auf Trockengewichtsbasis wirksame Bestandteile umfasst, wobei die wirksamen Bestandteile aus einem DPP-IV-Inhibitor, bei dem es sich um Vildagliptin oder ein pharmazeutisches Salz hiervon handelt, und Metformin oder in jedem Falle einem pharmazeutischen Salz hiervon bestehen, das folgende Stufen umfasst:
i) Granulieren des Metformins und eines Bindemittels,
ii) Trocknen das Metformin und das Bindemittel enthaltenden Granulats,
iii) Vermischen der DPP-IV-Inhibitor-Arzneimittelsubstanz, bei der es sich um Vildagliptin oder ein pharmazeutisches Salz hiervon handelt, mit dem Metformin und das Bindemittel enthaltenden Granulat,
iv) wobei optional ein Schmiermittel, z.B. Magnesiumstearat, mit dem in Stufe iii) erhaltenen Gemisch vermischt wird.
v) Verpressen der erhaltenen Mischung zur Bildung von Tabletten in Einheitsdosierungsform,
wobei es sich bei der Granulierung von Stufe i) um eine Schmelzgranulierung handelt.

2. Verfahren nach Anspruch 1, wobei während Stufe ii) das Granulat bis zu einem LOD von 0,5 bis 3,5 %, vorzugsweise von 1,5 bis 2,4 %, getrocknet wird.

3. Verfahren nach einem der Ansprüche 1 bis 2, wobei am Ende von Stufe ii) Metformin oder ein pharmazeutisches Salz hiervon in Form eines Granulats vorliegt, das zwischen 1 bis 25 Gew.-% oder zwischen 3 und 13 Gew.-% oder zwischen 4,9 und 12 Gew.-% oder zwischen 7,5 und 10,5 Gew.-% oder zwischen 7,5 und 17,5 Gew.-% oder zwischen 12,5 und 17,5 Gew.-% auf Trockengewichtsbasis eines pharmazeutisch akzeptablen Bindemittels umfasst.

4. Verfahren nach einem der Ansprüche 1 bis 3, wobei mindestens ein weiterer pharmazeutisch akzeptabler Hilfsstoff zu dem während Stufe i) oder während Stufe iii) zu vermischenden Gemisch zugegeben wird.

5. Verfahren nach Anspruch 4, wobei der weitere pharmazeutisch akzeptable Hilfsstoff ein Verdünnungsmittel oder ein Zerfallhilfsmittel ist.

6. Verfahren nach einem der Ansprüche 2 bis 5, wobei bei der bei Stufe v) erhaltenen Tablette eine weitere Beschichtungsstufe vorgenommen wird.

7. Verfahren nach einem der Ansprüche 1 bis 6, das eine Granulierstufe i), wobei Metformin und das Bindemittel gemischt werden und die Mischung durch einen Extruder für die Schmelzgranulierung geführt wird, umfasst.

8. Verfahren nach Anspruch 7, wobei der Extruder an der Mischzone auf zwischen 140 und 220 °C oder zwischen 155 und 205 °C oder zwischen 170 und 190 °C eingestellt ist.

9. Verfahren nach einem der Ansprüche 2 bis 8, wobei das Bindemittel eine Cellulose oder ein Derivat hiervon ist, die bzw. das aus mikrokristalliner Cellulose, Hydroxypropylcellulose, Hydroxyethylcellulose und Hydroxylpropylmethylcellulose ausgewählt ist.

## Revendications

1. Procédé pour la préparation d'un comprimé pharmaceutique comprenant entre 80 et 96 % en poids sur la base du poids sec d'ingrédients actifs, les ingrédients actifs étant constitués d'un inhibiteur de DPP-IV qui est la vildagliptine ou l'un de ses sels pharmaceutiques et de la metformine ou, dans l'un ou l'autre cas, l'un de leurs sels pharmaceutiques, qui comprend:
i) la granulation de la metformine et d'un liant,
ii) le séchage des granulés contenant la metformine et le liant,
iii) le mélange de l'inhibiteur de DPP-IV, de la substance médicamenteuse qui est la vildagliptine ou l'un de ses sels pharmaceutiques avec les granulés contenant la metformine et le liant,
iv) éventuellement un lubrifiant, par exemple, le stéarate de magnésium, qui est incorporé au mélange obtenu à l'étape iii),
v) la compression du mélange résultant pour former des comprimés sous une forme posologique unitaire, la granulation de l'étape i) étant une granulation par fusion.

2. Procédé selon' la revendication 1, dans lequel, lors de l'étape ii), les granulés sont séchés jusqu'à un LOD de 0,5-3,5%, de préférence, de 1,5-2,4%.

3. Procédé selon l'une quelconque des revendications 1 à 2, dans lequel à la fin de l'étape ii), la metformine ou l'un de ses sels pharmaceutiques est sous forme de granulés comprenant entre 1 et 25% ou entre 3 et 13% ou entre 4,9 et 12% ou entre 7,5 et 10,5%, ou entre 7,5 et 17,5% ou entre 12,5 et 17,5%, en poids sur la base du poids sec, d'un liant acceptable sur le plan pharmaceutique.

4. Procédé selon l'une quelconque des revendications 1 à 3, dans lequel au moins un excipient supplémentaire acceptable sur le plan pharmaceutique est ajouté au mélange à mélanger lors de l'étape i) ou lors de l'étape iii) .

5. Procédé selon la revendication 4, dans lequel l'excipient supplémentaire acceptable sur le plan pharmaceutique est un diluant ou un délitant.

6. Procédé selon l'une quelconque des revendications 2 à 5, dans lequel une étape d'enrobage supplémentaire est appliquée au comprimé résultant de l'étape v).

7. Procédé selon l'une quelconque des revendications 1 à 6, comprenant une étape de granulation i) dans laquelle la metformine et le liant sont mélangés et le mélange est passé à travers une extrudeuse pour la granulation par fusion.

8. Procédé selon la revendication 7, dans lequel l'extrudeuse est réglée à une température comprise entre 140 et 220°C, ou entre 155 et 205°C ou entre 170 et 190°C au niveau d'une zone de mélange.

9. Procédé selon l'une quelconque des revendications 2 à 8, dans lequel le liant est une cellulose ou l'un de ses dérivés, choisi parmi une cellulose microcristalline, une hydroxypropylcellulose, une hydroxyéthylcellulose et une hydroxypropylméthylcellulose.
